# EUROPÄISCHE PATENTSCHRIFT

(11) **EP 1 538 374 B1**
(45) Veröffentlichungstag und Bekanntmachung des Hinweises auf die Patenterteilung: **12.09.2007**
(21) Anmeldenummer: 04029053.8
(22) Anmeldetag: 08.12.2004
(51) Int. Cl.: F16J 15/06, H05K 5/06, A61M 5/178

(54) **Dichtungselement**
Sealing element
Elément d'étanchéité

(30) Priorität: 05.12.2003 DE 10356838
(43) Veröffentlichungstag der Anmeldung: 08.06.2005
(73) Patentinhaber: TecPharma Licensing AG, 3401 Burgdorf (CH)
(72) Erfinder: Hofstetter, Michael, 3052 Zollikofen (CH)

(56) Entgegenhaltungen:
- US-A- 5 334 799
- US-A- 5 822 192
- PATENT ABSTRACTS OF JAPAN Bd. 2003, Nr. 09, 3. September 2003 (2003-09-03) -& JP 2003 152346 A (SHIN ETSU POLYMER CO LTD), 23. Mai 2003 (2003-05-23)
- PATENT ABSTRACTS OF JAPAN Bd. 2003, Nr. 09, 3. September 2003 (2003-09-03) -& JP 2003 157740 A (SHIN ETSU POLYMER CO LTD), 30. Mai 2003 (2003-05-30)

## Beschreibung

Die vorliegende Erfindung bezieht sich auf ein Dichtungselement, insbesondere auf ein Dichtungselement für eine Verabreichungsvorrichtung zur dosierten Verabreichung medizinischer Substanzen, wie zum Beispiel eine Injektionsvorrichtung bzw. ein Pen.

Eine Ausführungsform einer bekannten Injektionsvorrichtung ist in der WO 03/053499 beschrieben.

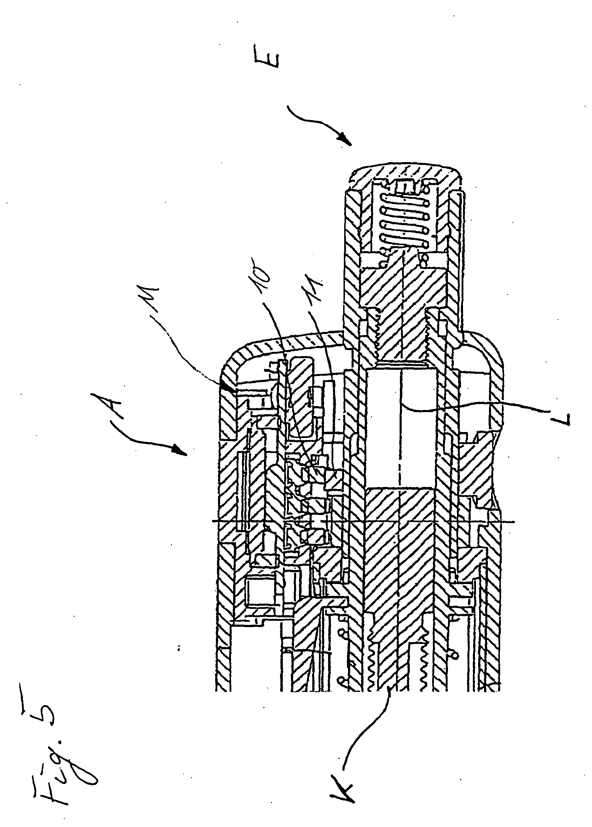

Figur 5 zeigt einen hinteren Teil eines bekannten Injektionsgeräts in einem Längsschnitt. Eine Kolbenstange K bewirkt den Vorschub eines Kolbens (nicht gezeigt) in die Vorschubrichtung (in Figur 5 nach links) auf einen nicht gezeigten Reservoirauslass zu entlang der Längsachse L, wobei die Größe der Verschiebung der Kolbenstange K durch eine Drehung des Einstellelements E festgelegt und durch Drücken des Einstellelements E ausgelöst werden kann. Um einem Benutzer anzuzeigen welche Dosis er durch Drehung des Einstellelements E eingestellt hat, ist ein Anzeigeelement A vorgesehen, auf welcher die Menge einer eingestellten Dosis dargestellt wird, wobei die eingestellte Dosis anhand der Bewegung von Kontaktelementen 10 ermittelt wird, welche durch eine oder mehrere Nockenscheiben 11 gedrückt werden, die durch eine Drehbewegung des Einstellelements E mitgedreht werden. Die Abfolge des Drückens und Freigebens der Kontaktelemente 10 durch die Nockenscheiben 11 ermöglicht es bei geeigneter Anordnung der Nockenscheiben 11 festzustellen um wie viel das Einstellelement E gedreht wurde, um eine durch die Drehung eingestellte abzugebende Dosis oder Wirkstoffmenge am Anzeigelement A anzuzeigen.

Jedoch kann durch für die Kontaktelemente 10 vorgesehenen Öffnungen zum Beispiel eine kleine Menge der von dem Pen abzugebenden Substanz in das sogenannte E-Modul M bzw. in die zur Auswertung der Bewegung der Kontaktelemente 10 vorgesehene Schaltung oder Leiterplatte eindringen und die Elektronik beschädigen. Ebenso kann es zu einer Beeinträchtigung der Elektronik bzw. der zum Betrieb der Elektronik vorgesehenen Batterie kommen, falls ein solcher Pen im Kühlschrank gelagert wird, um die optimale Lagerung der abzugebenden Substanz zu gewährleisten, wobei durch eine Abkühlung und ein Zusammenziehen der Luft leicht Feuchtigkeit in die Elektronik gelangen kann.

Aus JP2003157740 ist ein Dichtungselement aus nichtleitendem Gummi bekannt, wobei der elektrische Kontakt mit dem +/- Pol einer Batterie über leitende Drähte, die durch das Dichtelement hindurchgehen, hergestellt wird.

Es ist eine Aufgabe der vorliegenden Erfindung eine konstruktive Verbesserung einer Injektionsvorrichtung vorzuschlagen, durch welche die Ausfallsicherheit der Injektionsvorrichtung verbessert wird.

Diese Aufgabe wird durch den Gegenstand des unabhängigen Patentanspruchs gelöst. Vorteilhafte Ausführungsformen ergeben sich aus den abhängigen Ansprüchen.

Erfindungsgemäß wird vorgeschlagen ein Dichtungselement für eine Injektionsvorrichtung so auszugestalten, dass mit diesem Dichtungselement mindestens eine elektronische oder elektrische Komponente in der Verabreichungsvorrichtung bzw. dem Injektionsgerät abgedichtet werden kann, wobei eine oder mehrere Batterien durch das Dichtungselement abgedichtet werden. Besonders vorteilhaft wird eine für Kontaktelemente oder Druckstücke vorgesehene Kontaktmatte so vergrößert, dass diese Kontaktmatte als Dichtung für elektrische oder elektronische Komponenten und zur Abdichtung einer Batterie verwendet werden kann.

Unter dem Begriff "Dichtung" soll im Sinne der Erfindung jedes Element verstanden werden, welches allein oder in Kombination mit einem oder mehreren anderen Elementen, wie zum Beispiel einem Gehäuse und/oder einem Gehäusedeckel, verhindern kann, dass eine Flüssigkeit oder ein Gas wie z. B. Luft in einen bestimmten Raum eindringen kann, so dass in diesem Raum zum Beispiel elektrische oder elektronische Komponenten vor von außen einwirkenden Substanzen geschützt sind.

Das erfindungsgemäße Dichtungselement ist bevorzugt einstückig bzw. einteilig, also aus einem einzigen Element ausgebildet, in welchem z. B. abgetrennte Räume vorgesehen sind, in welchen zu schützende Komponenten vorgesehen sein können, wobei zum Beispiel das Dichtungselement selbst vollständig oder zumindest zum Teil, zum Beispiel an einer Oberfläche davon, leitend ausgebildet sein kann, um eine Wechselwirkung der von dem Dichtungselement eingeschlossenen Komponente mit der Umgebung zu ermöglichen. Ebenso kann das Dichtungselement elastisch sein, um zum Beispiel einen externen Druck auf eine von dem Dichtungselement umschlossene Komponente zu übertragen und hierdurch zum Beispiel ein Kontaktsignal herzustellen.

Das erfindungsgemäße Dichtungselement kann auch mit einem oder mehreren einteiligen oder mehrteiligen Gehäuseelementen zusammenwirken, um beispielsweise Komponenten abzudichten, welche von dem Gehäuse zum Beispiel nur zum Teil umschlossen sind, um die in dem Gehäuse angeordneten Komponenten zu schützen. Dabei kann ein Gehäuse ebenso wie das Dichtungselement starr oder elastisch und zum Beispiel mit einer leitenden Eigenschaft versehen sein, so dass von dem Gehäuse und/oder dem Dichtungselement abgedichtete Komponenten in Wechselwirkung mit der Umgebung oder externen Elementen treten können. Das Gehäuse kann zum Beispiel ein sogenannter Modulboden sein, an welchem als Dichtungselement zum Beispiel eine Kontaktmatte befestigt ist, um so eine Batterie und/oder eine Leiterplatte zu umschließen. Dabei kann das Gehäuse bzw. der Modulboden ein oder mehrere Rastelemente zum Beispiel in Form von Rastnasen aufweisen, um beispielsweise ein Modulfenster aufsetzen und fest mit dem Modulboden verbinden zu können, um eine oder mehrere Komponenten vollständig abdichten zu können. Ebenso kann das Gehäuse oder der Modulboden eine oder mehrere Öffnungen für Druckstücke bzw. Kontaktelemente aufweisen.

Wird das Dichtungselement so ausgestaltet, dass ein Batteriefach abgedichtet oder integriert werden kann, so kann die Batterie vor externen Substanzen, wie zum Beispiel ätzenden Flüssigkeiten, geschützt werden und bei geeigneter Auslegung des Dichtungselements, welches beispielsweise die Leiterplatte einer elektrischen Schaltung umschließt, kann die Batterie durch das Dichtungselement direkt in Kontakt mit der Leiterplatte gebracht werden, wodurch eine aufwändige Anbringung der Batterie zum Beispiel durch Löten und das Vorsehen von Kontaktbügeln entfallen kann, was den Herstellungsprozess deutlich vereinfacht und verbilligt und auch zu einer geringeren Störanfälligkeit führt.

Bevorzugt ist in dem Dichtungselement eine Ausnehmung oder Aussparung für die Batterie in Kombination mit einem leitenden Kontaktelement, wie zum Beispiel einem Graphitfass, vorgesehen, um Bereiche der Batterie, welche nicht unmittelbar auf einer Leiterplatte aufliegen, in elektrischen Kontakt mit der Leiterplatte bringen zu können.

Vorteilhaft ist mindestens ein Abtastelement, wie zum Beispiel eine oder mehrere Graphitpillen, in Verbindung mit dem Dichtungselement vorgesehen, um zum Beispiel bei einem externen Druck auf das Dichtungselement, zum Beispiel durch einen Nockenring, eine Verformung des Dichtungselements zu bewirken und hierdurch ein Abtastelement bzw. eine Graphitpille auf eine Leiterplatte zu drücken, wodurch ein elektrischer Kontakt auf der Leiterplatte hergestellt wird, durch welchen ein von einer Nockenscheibe erzeugtes Signal erfasst und ausgewertet werden kann.

Bevorzugt ist mindestens ein Entlüftungskanal in dem Dichtungselement vorgesehen, so dass sich das mindestens eine Abtastelement leicht hin und her bewegen kann ohne durch einen anliegenden Druck oder Sog in einer Position gehalten zu werden.

Das erfindungsgemäße Dichtungselement ist bevorzugt aus einem elastischen oder biegbaren Material, wie zum Beispiel aus einem geeigneten Kunststoff, Silikon oder Gummi gefertigt und kann ganz oder zumindest zum Teil, zum Beispiel an bestimmten Oberflächenbereichen, leitend sein, wodurch beispielsweise ein elektrischer Kontakt mit den Polen einer Batterie oder zu externen Komponenten oder in einer durch das Dichtungselement abgedichteten Schaltung oder Leiterplatte hergestellt werden kann.

Die Erfindung wird nachfolgend anhand eines bevorzugten Ausführungsbeispiels beschrieben. Es zeigen:
- Figur 1: einen Modulboden;
- Figur 2: eine auf den Modulboden als Dichtungselement aufgelegte Silikonmatte mit Graphitpillen und Graphitfass;
- Figur 3: eine auf das in Figur 2 gezeigte Dichtungselement aufgesetzte Leiterplatte;
- Figur 4: ein auf die in Figur 3 gezeigte Vorrichtung aufgesetztes Modulfenster; und
- Figur 5: einen Längsschnitt eines hinteren Teils eines Injektionsgerätes.

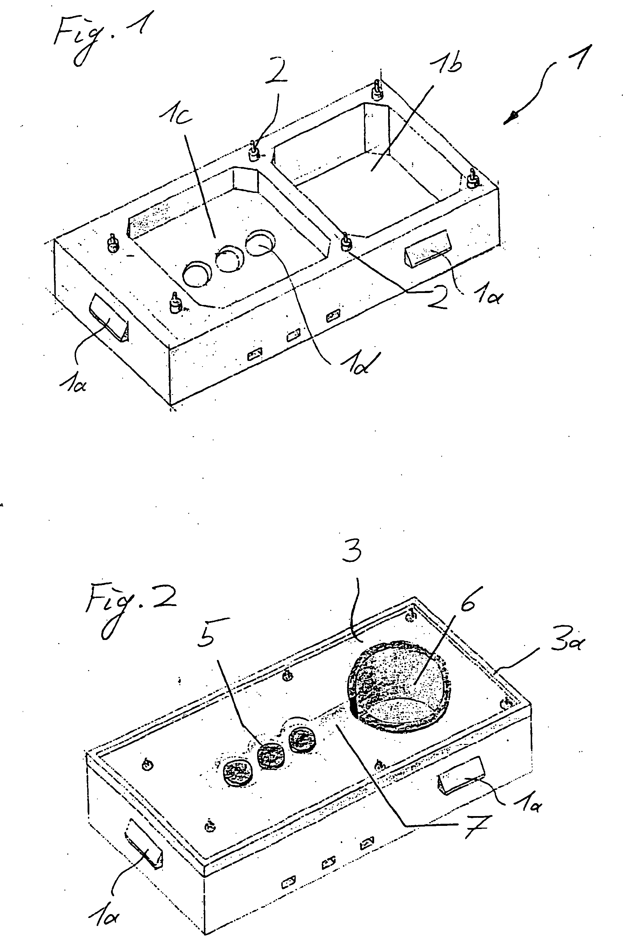

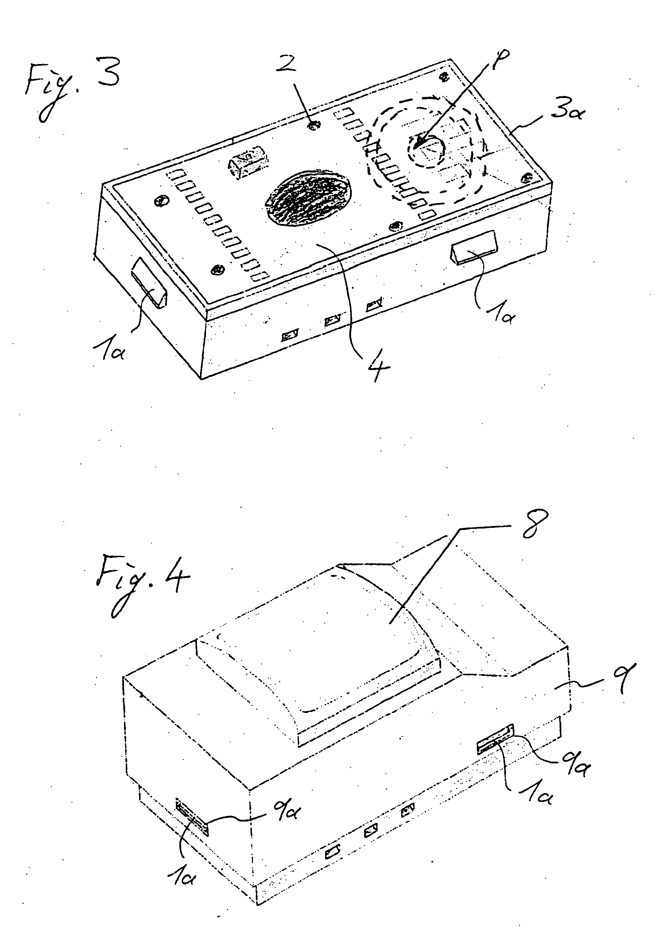

Figur 1 zeigt einen als Gehäuse dienenden Modulboden 1, an dessen Außenseite Rastnasen 1a zur Verrastung mit einem in Figur 4 gezeigten aufzusetzenden Modulfenstergehäuse 9 angebracht sind. Der Modulboden 1 weist in seinem Inneren zwei Fächer oder Räume 1b und 1c auf, wobei beispielsweise in den Raum 1b eine Batterie eingesetzt werden kann und der Raum 1c zur Übertragung von Druckbewegungen durch Kontaktelemente bzw. Druckstücke 10 durch in dem Boden des Moduls 1 vorgesehene Löcher 1d dient. An der Oberseite des Modulbodens 1 sind Distanzbolzen 2 vorgesehen, auf welche, wie in den Figuren 2 und 3 gezeigt, ein Dichtungselement 3 und eine Leiterplatte 4 aufgesetzt werden können.

Figur 2 zeigt den in Figur 1 gezeigten Modulboden mit darauf aufgesetzter Silikonmatte als Dichtungselement 3, wobei die Silikonmatte 3 den unteren größeren Außendurchmessern der Distanzbolzen 2 entsprechende Löcher aufweist, so dass die Silikonmatte 3 unverschiebbar durch die Distanzbolzen 2 auf dem Modulboden 1 positioniert ist. Die Silikonmatte 3 weist einen von der Oberfläche der Silikonmatte 3 hervorstehenden Rand 3a auf, so dass durch die Oberfläche der Silikonmatte 3 und den Rand 3a ein Bereich festgelegt wird, in welchen eine Leiterplatte 4 eingebracht werden kann. Die Silikonmatte 3 ist aus einem elastischen nicht leitendem Material gefertigt und hat an ihrer Oberseite Graphitpillen 5, welche Kontaktstellen der nachfolgend aufzubringenden Leiterplatte 4 gegenüberliegen, so dass ein von einem Nockenring 11 durch eine Öffnung 1d des Modulbodens 1 hindurch geschobenes Druckstück 10 auf die Unterseite der Silikonmatte 3 drückt und diese so verformt, dass eine auf der Oberseite der Silikonmatte 3 vorgesehene Graphitpille 5 nach oben gedrückt wird, um einen Kontakt auf der Leiterplatte 4 herzustellen.

Weiterhin ist in der Silikonmatte 3 eine Aussparung für ein Batteriefach vorgesehen, in welches ein Graphitfass 6 eingesetzt wird, um einen elektrisch leitenden Kontakt mit einer Außenseite und gegebenenfalls einer Unterseite der Batterie herzustellen. Zwischen dem Batteriefach und den Graphitpillen 5 ist ein Entlüftungskanal 7 vorgesehen, so dass die Graphitpillen 5 einfach hin und her bewegt werden können ohne durch Druck- oder Sogeffekte in ihrer freien Bewegung behindert zu werden. Ebenso erleichtert der Entlüftungskanal 7 das Einsetzen bzw. Herausnehmen einer Batterie in das bzw. aus dem Batteriefach.

Figur 3 zeigt die in Figur 2 gezeigte Vorrichtung, auf welche eine Leiterplatte 4 mit einem geschwärzt eingezeichneten Chip aufgesetzt ist. Die Distanzbolzen 2 haben im oberen Bereich jeweils ein Teilstück mit kleinerem Außendurchmesser, welcher in etwa Löchern in der Leiterplatte 4 entspricht, so dass die Leiterplatte 4 durch die oberen Teilbereiche der Distanzbolzen 2 positioniert wird. Die Leiterplatte 4 ist vollständig vom Rand 3a der Silikonmatte 3 umgeben, wodurch einerseits die Leiterplatte 4 vor einem seitlichen Eindringen von Substanzen geschützt wird und andererseits die unterhalb der Leiterplatte 4 angeordnete in das Graphitfass 6 eingesetzte Batterie vollständig abgedichtet ist. Die Batterie kontaktiert über einen an der Batterie vorgesehenen Kontakt, welcher unmittelbar auf der Unterseite der Leiterplatte 4 aufliegt, eine auf der Unterseite der Leiterplatte 4 im Bereich des Pfeils P vorgesehene Elektrode. Die Außenseite der Batterie steht über das Graphitfass 6 mit einer ebenfalls auf der Unterseite der Leiterplatte 4 vorgesehenen in etwa ringförmigen gestrichelt eingezeichneten Elektrode in Verbindung, so dass der + und -Pol der Batterie ohne Lötarbeiten und ohne Kontaktbügel durch einen einfachen Herstellungsprozess in elektrische Verbindung mit der Leiterplatte 4 gebracht werden können.

Die Distanzbolzen 2 können anschließend zum Beispiel durch Ultraschall angeschmolzen werden und so die Leiterplatte 4 auf dem Modulboden 1 fixieren, so dass die Batterie durch die Silikonmatte 3 an die Unterseite der Leiterplatte 4 gepresst wird. Nachfolgend kann die gesamte Baugruppe programmiert und zum Beispiel auf einen gewünschten Zählschritt konfiguriert werden.

Figur 4 zeigt die in Figur 3 gezeigte Baugruppe mit einem aufgesetzten Modulfenstergehäuse 9, welches Ausnehmungen 9a aufweist, in welche die Rastnasen 1a des Modulbodens 1 einrasten können, um so das Modulfenstergehäuse 9 fest mit dem Modulboden 1 zu verbinden und so eine sichere Abdichtung zu gewährleisten. Das Modulfenstergehäuse 9 weist ein Modulfenster 8 auf, welches zumindest zum Teil durchsichtig ist und unter welchem zum Beispiel eine auf der Leiterplatte 4 vorgesehene LCD-Anzeige (in Figur 3 nicht gezeigt) vorgesehen sein kann.

Somit kann mittels des Modulbodens 1 und des Modulfenstergehäuses 9 die gesamte Leiterplatte 4 einschließlich der Batterie umschlossen und über die Silikonmatte 3 abgedichtet werden, wobei der elektrische Kontakt zwischen der Batterie und der Leiterplatte 4 ohne einen zusätzlichen Kontaktbügel sichergestellt werden kann. Dies ermöglicht es die Bauhöhe der gesamten Baugruppe zu reduzieren, wobei beispielsweise der so gewonnene Platz für eine weitere Abdichtung verwendet werden kann. Somit kann die Batterie ohne Lötstellen im Modul befestigt werden, wodurch kein schädliches Blei mehr zur Herstellung des Moduls verwendet wird.

## Patentansprüche

1. Injektionsgerät mit einem Modul, das ein Gehäuseelement und in dem Gehäuseelement einen Raum für die Aufnahme von elektrischen Komponenten besitzt, wobei ein Dichtungselement (3) vorhanden und so ausgebildet ist, dass es den Raum für die Aufnahme der elektrischen Komponenten nach Aussen abdeckt und abdichtet, **dadurch gekennzeichnet, dass** das Dichtungselement (3) zumindest zum Teil an bestimmten Oberflächenbereichen leitende Bereiche aufweist, wodurch ein elektrischer Kontakt mit dem +/- Pol einer Batterie hergestellt wird.

2. Injektionsgerät nach Anspruch 1, **dadurch gekennzeichnet, dass** das Dichtungselement (3) einteilig ist.

3. Injektionsgerät nach einem der vorhergehenden Ansprüche, **dadurch gekennzeichnet, dass** die elektrischen Komponenten eine Leiterplatte (4), ein Chip und/oder eine Batterie sind.

4. Injektionsgerät nach einem der vorhergehenden Ansprüche, **dadurch gekennzeichnet, dass** die leitenden Bereiche ein einsetzbares Kontaktelement (6) sind.

5. Injektionsgerät nach einem der vorhergehenden Ansprüche, **dadurch gekennzeichnet, dass** das Dichtungselement (3) ein leitfähiges Abtastelement (5) besitzt.

6. Injektionsgerät nach einem der zwei vorhergehenden Ansprüche mit einem Entlüftungskanal (7), welcher in Verbindung mit mindestens einem Abtastelement (5) und/oder der Ausnehmung für die Batterie vorgesehen ist.

7. Injektionsgerät nach einem der vorhergehenden Ansprüche, **dadurch gekennzeichnet, dass** das Dichtungselement (3) aus einem elastischen Material gebildet ist.

## Claims

1. Injection device with a module, which has a housing element and in the housing element a space for accommodating electrical components, a sealing element (3) being present and being formed in such a way that it covers and seals externally the space for accommodating the electrical components, **characterized in that** the sealing element (3) has conductive areas at least in part on certain surface areas, due to which an electrical contact with the +/- pole of a battery is produced.

2. Injection device according to claim 1, **characterized in that** the sealing element (3) is in one piece.

3. Injection device according to one of the preceding claims, **characterized in that** the electrical components are a printed circuit board (4), a chip and/or a battery.

4. Injection device according to one of the preceding claims, **characterized in that** the conductive areas are an insertable contact element (6).

5. Injection device according to one of the preceding claims, **characterized in that** the sealing element (3) has a conductive sensing element (5).

6. Injection device according to one of the two preceding claims with a vent channel (7), which is provided in connection with at least one sensing element (5) and/or the recess for the battery.

7. Injection device according to one of the preceding claims, **characterized in that** the sealing element (3) is formed from an elastic material.

## Revendications

1. Appareil d'injection comportant un module qui possède un élément de logement et, dans l'élément de logement, un espace pour la réception de composants électriques, un élément d'étanchéité (3) étant présent et conçu de telle sorte qu'il recouvre et étanchéifie vers l'extérieur l'espace pour la réception des composants électriques, et **caractérisé en ce que** l'élément d'étanchéité (3) présente au moins en partie, sur des zones de surface déterminées, des zones conductrices, permettant d'établir un contact électrique avec le pôle +/- d'une batterie.

2. Appareil d'injection selon la revendication 1, **caractérisé en ce que** l'élément d'étanchéité (3) est d'une seule pièce.

3. Appareil d'injection selon l'une des revendications précédentes, **caractérisé en ce que** les composants électriques sont une plaque conductrice (4), une puce et/ou une batterie.

4. Appareil d'injection selon l'une des revendications précédentes, **caractérisé en ce que** les zones conductrices sont un élément de contact (6) pouvant être mis en oeuvre.

5. Appareil d'injection selon l'une des revendications précédentes, **caractérisé en ce que** l'élément d'étanchéité (3) possède un élément palpeur conducteur (5).

6. Appareil d'injection selon l'une des deux revendications précédentes, comportant un conduit de ventilation (7) qui est prévu pour être en liaison avec au moins un élément palpeur (5) et/ou l'évidement pour la batterie.

7. Appareil d'injection selon l'une des revendications précédentes, **caractérisé en ce que** l'élément d'injection (3) est formé par un matériau élastique.
